Europäisches Patentamt

European Patent Office

Office européen des brevets

(19)

(11) EP 0 966 960 A1

# EUROPEAN PATENT APPLICATION

(12)

(43) Date of publication:
29.12.1999  Bulletin 1999/52

(51) Int. Cl.$^6$: **A61K 31/045**

(21) Application number: 98109458.4

(22) Date of filing: 25.05.1998

(84) Designated Contracting States:
**AT BE CH CY DE DK ES FI FR GB GR IE IT LI LU MC NL PT SE**
Designated Extension States:
**AL LT LV MK RO SI**

(71) Applicant:
**DAINIPPON INK AND CHEMICALS, INC.**
**Itabashi-ku Tokyo (JP)**

(72) Inventors:
• **Kato, Toshimitsu**
  **Chiba-ken (JP)**

• **Nishino, Hoyoku**
  **Osaka (JP)**
• **Saiki, Ikuo**
  **Toyama-ken (JP)**

(74) Representative:
**Kraus, Walter, Dr. et al**
**Patentanwälte Kraus, Weisert & Partner**
**Thomas-Wimmer-Ring 15**
**80539 München (DE)**

(54) **Agent for inhibiting carcinogenesis or tumor metastasis**

(57)     In order to provide a naturally derived agent for inhibiting carcinogenesis or tumor metastasis, having a low toxicity and excellent effectiveness in inhibiting carcinogenesis and tumor metastasis, zeaxanthin, and particularly zeaxanthin which is extracted from an alga, is used as an active ingredient.

EP 0 966 960 A1

**Description**

BACKGROUND OF THE INVENTION

1. FIELD OF THE INVENTION

The present invention relates to an agent for inhibiting carcinogenesis or tumor metastasis, the agent comprising zeaxanthin as an active ingredient.

This application is based on patent applications Nos. Hei 8-316169 and Hei 9-122146, both filed in Japan, the contents of which are incorporated herein by reference.

2. BACKGROUND ART

As methods for treating cancer, surgical treatments, radiotherapy, chemotherapy (administration of a medicament), and the like are presently conducted in general. As chemotherapies, treating methods have been hitherto widely employed in which a medicament is administered which directly acts on tumor cells and kills the tumor cells; various anti-tumor agents for use in these types of therapies have been proposed. However, such medicaments which act on tumor cells not only kills tumor cells but also acts on normal cells. Therefore, medicaments used in chemotherapy have a shortcoming in that they have strong side effects, although such medicaments are highly effective in treating cancer.

In addition, although the above methods are effective means for removing or killing a primary focus of the cancer, since minute metastasis to another tissue or organ has been already occurred in most cases when the primary focus is found, it is extremely difficult to completely cure or exterminate the cancer. Furthermore, in chemotherapies, administration of a great amount of such a medicament causes serious side effects in many cases, overcoming which has become an important objective in the treatment of cancer.

Therefore, as new anti-tumor agents, medicaments which indirectly inhibit proliferation of tumor cells by enhancing immunocompetence of normal cells have been attracting attention; for example, crestin, interferon, etc., have been developed. These medicaments have advantages in having few side effects, although their effect on tumor cells is weak. Furthermore, it has been pointed out that carotenoids are effective in inhibiting proliferation of tumor cells; for example, *Oncology*, vol. 39, pp. 33-37, (1982), and *Biochemical and Biophysical Research Communication*, pp. 1130-1135, (1986), report on β-carotene; Japanese Patent Application, First Publication (Kokai), No. Hei 1-104009 reports on α-carotene; and Japanese Patent Application, First Publication (Kokai), No. Hei 6-227970 reports on lycopene.

On the other hand, various studies have been conducted on physiological effects of algae, particularly cyanobacteria (cyanophyceae), and more particularly spirulina which is used as a foodstuff; for example, studies are known which were conducted on serum cholesterol lowering effects (*Nutr. Report Int.*, vol. 28, p. 519, (1983); *Nippon Eiyo Shokuryo Gakkai Shi* (Journal of the Japanese Society of Nutritional and Food Science) vol. 37, p. 323, (1984); and *Nutr. Report Int.*, vol. 37, p. 1329, (1988); blood pressure reducing effects (*Joshi Eiyo Daigaku Kiyo* (The Bulletin of Kagawa Nutrition University), vol. 21, p. 63, (1990)); intestinal microflora improving effects (*Chiba Kenritsu Eisei Tanki Daigaku Kiyo* (The Bulletin of Chiba Prefectural College of Hygiene), vol. 5, No. 2, p. 27, (1987)); effects of inhibiting functional disorder of kidneys (The 108th, 109th, and 110th Meetings of the Japanese Society of Pharmaceutics in 1988, 1989, and 1990); alcohol metabolism improving effects (*Nippon Eiyo Shokuryo Gakkai Shi* (Journal of the Japanese Society of Nutritional and Food Science) vol. 47, p. 395, (1994)); immunological activation effects (*J. Nutr. Sci. Vitaminol.*, vol. 40, p. 431, (1994)); and anti-viral effects (*Phytotherapy Research*, vol. 7, p. 76, (1993)).

In particular, cancer-inhibiting agents comprising spirulina of cyanobacteria as a main ingredient are disclosed in Japanese Patent Application, First Publication (Kokai), No. Sho 54-73108. According to this publication, water-soluble substances in spirulina are utilized. Therefore, the cancer-inhibiting agents in the publication differ in the active ingredients and in the action mechanism from the agents for inhibiting carcinogenesis according to the invention described in the present application. As stated above, β-carotene, α-carotene, and lycopene are known as carotenoid substances which inhibit carcinogenesis; however, there are also some reports that on the contrary β-carotene, in particular, promotes lung cancer, and therefore, development of a carcinogenesis-inhibiting substance which has a low toxicity and is more effective is hoped for.

SUMMARY OF THE INVENTION

The object to be attained by the present invention is to provide a naturally derived agent for inhibiting carcinogenesis or tumor metastasis, the agent having a low toxicity and excellent effectiveness in inhibiting carcinogenesis and tumor metastasis.

The inventors have diligently conducted examination of substances having activity in inhibiting carcinogenesis or in inhibiting metastasis of tumors, in particular, substances having anti-tumor promoter activity, among carotenoids which exist in nature and particularly in food. As a result, the inventors have found that from among such substances, zeaxanthin, in particular, has strong effects in inhibiting carcinogenesis and in inhibiting metastasis of tumor, and the present invention has been attained based on these findings. That is, the present invention is:

(1) Zeaxanthin for use as an active ingredient in a medicament to inhibit carcinogenesis.
(2) Zeaxanthin according to the above description (1), wherein zeaxanthin is extracted from an alga.
(3) Zeaxanthin according to the above description (2), wherein the alga is cyanobacterium.
(4) Zeaxanthin according to the above description (3), wherein the cyanobacterium is an alga belonging to the genus *Spirulina*.
(5) Use of the zeaxanthin of any one of the above descriptions (1)~(4) for the manufacture of a medicament to inhibit carcinogenesis.
(6) Use of the zeaxanthin of any one of the above descriptions (1)~(4) as an active ingredient for inhibiting carcinogenesis.
(7) An agent for inhibiting carcinogenesis, the agent comprising the zeaxanthin of any one of the above descriptions (1)~(4).
(8) Use of the agent of the above description (7) for inhibiting carcinogenesis.
(9) Zeaxanthin for use as an active ingredient in a medicament to inhibit metastasis of tumor.
(10) Zeaxanthin according to the above description (9), wherein zeaxanthin is extracted from an alga.
(11) Zeaxanthin according to the above description (10), wherein the alga is cyanobacterium.
(12) Zeaxanthin according to the above description (11), wherein the cyanobacterium is an alga belonging to the genus *Spirulina*.
(13) Use of the zeaxanthin of any one of the above descriptions (9)~(12) for the manufacture of a medicament to inhibit metastasis of tumor.
(14) Use of the zeaxanthin of any one of the above descriptions (9)~(12) as an active ingredient for inhibiting metastasis of tumor.
(15) An agent for inhibiting metastasis of tumor, the agent comprising the zeaxanthin of any one of the above descriptions (9)~(12).
(16) Use of the agent of the above description (15) for inhibiting carcinogenesis.

According to the present invention, a naturally derived agent for inhibiting carcinogenesis or tumor metastasis can be provided, the agent having a low toxicity and excellent effectiveness in inhibiting carcinogenesis and tumor metastasis.

## DESCRIPTION OF THE PREFERRED EMBODIMENTS

It is known that higher plants (corn, alfalfa, etc.) and algae (cyanophyceae, chlorophyceae, rhodophyceae, etc.) contain zeaxanthin, which can be used in the present invention. Among these, cyanophyceae contains a great amount of zeaxanthin. Examples of genera in cyanophyceae are the genus *Spirulina*, the genus *Fischerella*, the genus *Anabaena*, the genus *Nostoc*, the genus *Cynechocystis*, the genus *Cynechococcus*, and the genus *Tolypothrix*.
Among these, an alga which belongs to the genus *Spirulina* is most preferable as a material for zeaxanthin since such an alga is produced on an industrial scale, and is used for food, and therefore, the safety thereof is ensured. In addition, as an agent for inhibiting carcinogenesis and tumor metastasis, using zeaxanthin having little impurity obtained by extracting zeaxanthin from an alga and by refining it is more preferable than using an alga by itself, and using zeaxanthin having a purity of 95% or higher is even more preferable.
The zeaxanthin to be used in the present invention may be a raw extract which is extracted from the aforementioned higher plants or algae by an extraction method using an alcohol (such as ethanol and methanol), a ketone (such as acetone), chloroform, an ether, or an ester, or by a supercritical fluid extraction method using carbon dioxide. In addition, refined zeaxanthin which is obtained by further refining the raw extract may also be used. One hundred grams of alga of the genus *Spirulina* normally contains 50-150 mg zeaxanthin. Since zeaxanthin is susceptible to oxidation by air or to photolysis, it is preferable that zeaxanthin be handled and stored in a cool dark place in an atmosphere of an inert gas.
An agent for inhibiting carcinogenesis or tumor metastasis according to the present invention may be administered orally or non-orally (for example, intravenous administration or the like). The agent to be administered may be prepared in the form of a pharmaceutical product suitable for the relevant method of administration. Such a pharmaceutical product may be prepared in the form of a tablet, a capsule, granules, grains, powder, a troche, injection, emulsion, suspension, syrup, or the like, according to its utility. When preparing such a pharmaceutical product, non-toxic additives which are normally used in these types of products, such as an excipient, a binder, a disintegrator, a lubricant, a preservative, an anti-oxidative agent, an isotonic agent, a buffering agent, a coating agent, a sweetening agent, dissolving agent, a base, a dispersing agent, a stabilizing agent, and a colorant, may be used, and the pharmaceutical product can be obtained by a known method. The amount of the active ingredient of the present invention contained in a pharmaceutical product may vary depending on the form of the product; however, in general, it is desirable that a pharmaceutical product contain the active ingredient in a concentration of 0.01~100% by weight.

The administered amount of an agent for inhibiting carcinogenesis or tumor metastasis according to the present invention may vary widely depending on the type of the subject of the administration (human and other warm-blooded animals), the condition of the patient, the doctor's diagnostic results, etc. In the case of oral administration, 0.5~50 mg, and preferably 0.5~20 mg, of zeaxanthin as an active ingredient for each 1 kg of weight of the subject per day may be administered. In the case of non-oral administration, 0.1~10 mg, and preferably 0.5~5 mg, of zeaxanthin as an active ingredient for each 1 kg of weight of the subject per day may be administered. In addition, the above amount may be administered as a single dose once per day or as a several doses separated in time during the day. Such methods of administration may arbitrarily vary depending on the condition of the patient, and on the doctor's diagnostic results.

Since an agent for inhibiting carcinogenesis or tumor metastasis containing zeaxanthin as an active ingredient according to the present invention manifests the effect of inhibiting cancer during the promotion stage and in an anti-oxidative manner, this agent is not selective about the types of tumor. Therefore, this agent is effective in prophylaxis of hepatoma, stomach cancer, cancer of the pancreas, uterocervical cancer, lung cancer, large intestine cancer, mammary cancer, encephaloma, leukemia, larynx cancer, cancer of the esophagus, etc.; in inhibition of metastasis of colon cancer to the liver; and in inhibition of metastasis of stomach cancer, cancer of the pancreas, uterocervical cancer, lung cancer, hepatoma, mammary cancer, encephaloma, leukemia, larynx cancer, cancer of the esophagus, etc., to another organ.

The higher the purity of the agent for inhibiting carcinogenesis and tumor metastasis containing zeaxanthin as an active ingredient according to the present invention, the better, in respect of ensuring the safety of the pharmaceutical product; however, since impurities which contaminate zeaxanthin are carotenoids (of which zeaxanthin is one), a purity of 90% or higher is, in general, sufficient for ensuring safety.

An agent for inhibiting carcinogenesis or tumor metastasis according to the present invention has an effect of preventing the natural occurrence of cancer, i.e., a carcinogenesis-inhibiting effect, and also has an effect of inhibiting metastasis of an exisiting cancer to another organ, and thereby the agent according to the present invention has an effect of assisting in the more effective performance of common methods of treating cancer such as surgical treatments and radiotherapy.

EXAMPLES

The present invention will be explained by way of examples; however, these examples are only some of the aspects of the present invention, and the present invention should not be limited to the examples.

Example of Production of Zeaxanthin

To 100 g of spirulina, 400 g of methanol was added, and zeaxanthin was extracted at 60~64°C overnight. The spirulina alga was filtered out in a nitrogen atmosphere pressurized at 0.1 kg/cm$^2$, 400 g of methanol was further added to the thus-obtained residue, and zeaxanthin was extracted at 60~64°C for 3 hours. The thus-obtained filtrate was added to the methanol extract which was obtained previously, and methanol in the thus-mixed methanol extract was removed by evaporation under a depressurized nitrogen atmosphere to obtain a concentrate having a solid content of 12.1 g.

To this concentrate, 40 g of methanol and 5g of KOH was added, and a saponification reaction was allowed to proceed at 64~65°C for 2 hours. After saponification, methanol was removed by evaporation, and the thus-obtained concentrate was dissolved in 50 g of water and 100 g of ethyl acetate which were added to the concentrate. Liquid-liquid extraction was conducted to obtain an ethyl acetate extract. The ethyl acetate in the ethyl acetate extract was removed by evaporation under depressurized atmosphere, and a solid content of 2.25 g was obtained as a carotenoid fraction. The carotenoid fraction was subjected to silica gel column chromatography (silica gel C-200, 50 g) using an eluate of n-hexane/ethyl acetate = 3/1 ~ 2/1 to separate zeaxanthin from the other carotenoids.

The thus-obtained fraction containing zeaxanthin was again subjected to silica gel column chromatography (silica gel C-200, 40 g) using an eluate of n-hexane/ethyl acetate = 1/1. The thus-obtained zeaxanthin fraction was concentrated under a depressurized nitrogen atmosphere to precipitate crystals. The crystals were filtered out and then dried in a vacuum at room temperature to obtain 47 mg zeaxanthin crystals.

The thus-obtained zeaxanthin crystals were analyzed by a high performance liquid chromatograph "LC-6A" manufactured by Shimadzu Corporation under the following conditions:

Column: "WAKOSIL 60-5", 4.6 mm × 150 mm

Detect wavelength: 450 nm

Temperature of column: 40°C

Mobile phase: n-hexane : acetone = 4:1 (v/v)

Flow rate: 1.0 ml/minute.

As a result of the analysis, the purity of zeaxanthin was found to be 98.7%. The following examples were conducted using zeaxanthin samples obtained in production tests in a scale hundred times larger than that of the above example of production of zeaxanthin.

Example 1 Carcinogenesis-inhibiting Effect with regard to Naturally Occurring Hepatoma

C3H/He female mice (control group: 14 mice; test group: 12 mice), in which hepatoma is known to occur naturally, were allowed to freely take food and water for 40 weeks. For the mice of the test group, 20 mg zeaxanthin (having a purity of 98.5%) which was obtained in a manner similar to that of the above example of production of zeaxanthin was suspended in 1 ml dimethyl sulfoxide (DMSO), and thereafter the suspension was added to the drinking water of the test so that the final concentration of zeaxanthin was 0.005%. On the other hand, for the control group, only DMSO in the same amount was added to the drinking water. At the end of the 40 weeks, both groups of mice were sacrificed, and the number of tumors which occurred in the liver was counted. The results are shown in Table 1.

Table 1

| | Number of mice | Number of mice in which tumor occurred | Average number of tumors |
|---|---|---|---|
| Control Group | 14 | 5 (35.7%) | 1.75 |
| Test group | 12 | 1 ( 8.3%) | 0.08 |

It is clear from the results shown in Table 1 that the number of mice in which tumors occurred and the average number of tumors per mouse were restricted in the test group (inhibition rate: 95%); the effect of zeaxanthin in inhibiting natural carcinogenesis is clear. On the other hand, β-carotene has been found to have no such effect according to experiments conducted in the same manner as in Example 1.

Example 2 Effect of Inhibiting Metastasis of Colon Cancer to Liver

The abdomen of a BALB/c mouse was sectioned, and $2 \times 10^4$ cells of a colon cancer strain highly metastasizable to liver (MOUSE COLON 26) were injected into the portal vein. Zeaxanthin (4 μg/mouse in Test Example 1; 40 μg/mouse in Test Example 2; 400 μg/mouse in Test Example 3) and all-trans retinoic acid (ATRA) as a positive control were orally administered everyday for 5 consecutive days after the inoculation with tumor cells and then the administration was discontinued for 2 days. The cycle of the 5-day administration and the 2-day discontinuation was repeated twice. Twenty days later, the mouse was sacrificed, and the number of visible tumor tubers in the liver was counted. The results are shown in Table 2. In Table 2, the mark "*" indicates a significant difference when $p < 0.005$ (Whitney's U-test).

Table 2

| | Number of mice | Number of visible tumor tubers (average ± SD) | Average weight of liver (g) |
|---|---|---|---|
| Comparative Example (no administration) | 8 | 12.25 ± 6.13 | 2.16 |
| Test Example 1 | 7 | 6.86 ± 2.27 | 1.36 |
| Test Example 2 | 8 | 1.87 ± 1.96* | 0.99 |
| Test Example 3 | 8 | 1.00 ± 2.13* | 0.99 |
| Example of administration of ATRA | 6 | 0.83 ± 1.17* | 0.92 |
| SD: standard deviation | | | |

As is clear from the results in Table 2, in the group of the mice to which zeaxanthin was administered, the number of visible tumor tubers decreased dependently on the dosage; zeaxanthin evidently inhibited metastasis of colon cancer to the liver. In addition, increase in the weight of the liver, which is observed when a tumor is metastasized to the liver, was also inhibited. Similar results were also obtained with regard to the group of mice to which ATRA was administered.

Example 3 Effect of Inhibiting Invasion of Basement Membrane

The action mechanism of zeaxanthin for inhibiting metastasis of colon cancer to the liver in Example 2 was examined using a Transwell chamber.

A chamber which is separated into two layers (upper and lower layers) by a membrane filter having pores of 8 $\mu$m in diameter was used, the upper surface of the filter being coated with 5 $\mu$g of matrigel (a substrate for restructuring a basement membrane), and the lower surface being coated with 1 $\mu$g of fibronectin. B16-BL6 melanoma cells having a cell density of $2 \times 10^5/\mu l$ were added to the upper layer of the chamber, and were incubated in a 5%-$CO_2$ incubator at 37°C for 4 hours in the presence of 0.05~500 $\mu$M of zeaxanthin. After the incubation, the number of cells which invaded the surface of the lower part of the filter and shifted thereto were counted using an optical microscope. The results are shown in Table 3. In Table 3, the Comparative Example shows the result of a test in which only DMSO, which was used in the preparation of the samples in the Test Examples, was used. In Table 3, the mark "*" indicates a significant difference when $p < 0.05$ (Whitney's U-test), and the mark "**" indicates that of $p < 0.005$.

Table 3

| | Concentration of zeaxanthin ($\mu$M) | Number of cells which invaded/shifted to basement membrane |
|---|---|---|
| Comparative Example | 0 | $157 \pm 21$ |
| Test Example 4 | 0.05 | $137 \pm 17$ |
| Test Example 5 | 0.5 | $119 \pm 17$ |
| Test Example 6 | 5 | $109 \pm 12$* |
| Test Example 7 | 50 | $88 \pm 7$** |
| Test Example 8 | 500 | $36 \pm 12$** |

As is clear from the results in Table 3, zeaxanthin inhibited melanoma cells from invading the basement membrane in accordance with the concentration of zeaxanthin. These results suggest that one of the action mechanisms of zeaxanthin for inhibiting metastasis of colon cancer to liver is inhibition of cancer cells from invading a basement membrane.

Example 4 Effect of Inhibiting Carcinogenesis (Lung)

As a carcinogenesis initiator, 4-nitroquinoline-1-oxide dissolved in a solution of olive oil and cholesterol mixed in the ratio of 20:1 was administered subcutaneously in a single dose to ddY mice (a control group of 15 mice and a test group of 15 mice) in an amount of 10 mg/3.3ml/kg weight. Four weeks after administration, an 8% water solution of glycerin as a carcinogenesis promoter was given to the mice of the control group as drinking water for 25 weeks in a manner such that they could take it freely. (The duration of carcinogenesis promotion was 25 weeks).

In a manner similar to that for the control group, the carcinogenesis initiator was subcutaneously administered to the mice of the test group, and 4 weeks after the administration, an 8% water solution of glycerin as a carcinogenesis promoter was given to the mice of the test group as drinking water for 25 weeks in a manner such that they could take it freely, while zeaxanthin suspended in a liquid mixture of olive oil and "Tween 80" was forcibly administered to the mice of the test group 3 times a week throughout the 25-week duration of carcinogenesis promotion in an amount of 0.2 g per mouse per administration.

In addition, food and drinking water was given to the mice in a manner such that they could take it freely. Twenty five weeks after the start of the administration of zeaxanthin, that is, after the duration of carcinogenesis promotion was completed, the mice of both the control group and the test group were sacrificed, and the number of mice in which lung cancer occurred, and the average number of tumors were counted. The results are shown in Table 4.

In Table 4, those values in parentheses indicate values obtained by dividing the number of mice in each group in which lung cancer occurred by the total number of mice in each group. The average number of tumors was obtained by dividing the total number of tumors in all mice in each group by the total number of mice in each group.

Table 4

|  | Number of mice | Number of mice in which lung cancer occurred | Average number of tumors |
|---|---|---|---|
| Control Group | 15 | 11 (73%) | 1.40 |
| Test Group | 15 | 5 (33%) | 0.38 |

The inhibition ratio of carcinogenesis of the test group was determined as follows:

$$\text{Inhibition ratio of carcinogenesis} = \{(1.40 - 0.38) / 1.40\} \times 100 = 73\%$$

It is clear that the number of mice in which lung cancer occurred and the average number of tumors were restricted in the test group; the effect of zeaxanthin in inhibiting occurrence of lung cancer, and, in particular, in inhibiting carcinogenesis promotion, is clear.

Example 5 Effect of Inhibiting Carcinogenesis (Skin)

The back of an ICR male mouse (control group: 15 mice; test group: 14 mice) was shaved, and 100 μg of 7,12-dimethylbenz[a]anthracene in 100 μl of acetone was applied thereto. Two days after the application, application of 1.6 nmol of 12-O-tetradecanoylphorbal-13-acetate (TPA) in 100 μl of acetone was begun and repeated twice a week. The duration of carcinogenesis promotion was set to 15 weeks.
An hour before every TPA application, 160 nmol zeaxanthin (which makes the molar ratio of TPA to zeaxanthin 1:100) in 100 μl of acetone was applied to the mice of test group. After the 15-week duration of carcinogenesis promotion, the ratio of occurrence of skin tumor and the average number of tumors were measured. The results are shown in Table 5.
In Table 5, those values in parentheses indicate values obtained by dividing the number of mice in each group in which skin cancer occurred by the total number of mice in each group. The average number of tumors was obtained by dividing the total number of tumors in all mice in each group by the total number of mice in each group.

Table 5

|  | Number of mice | Number of mice in which skin cancer occurred | Average number of tumors |
|---|---|---|---|
| Control Group | 15 | 7 (47%) | 1.60 |
| Test Group | 14 | 3 (21%) | 0.86 |

The inhibition ratio of carcinogenesis of the test group was determined as follows:

$$\text{Inhibition ratio of carcinogenesis} = \{(1.60 - 0.86) / 1.60\} \times 100 = 46\%$$

It is clear that the number of mice in which skin cancer occurred and the average number of tumors were restricted in the test group; the effect of zeaxanthin in inhibiting occurrence of skin cancer, and, in particular, in inhibiting carcinogenesis promotion, is clear.

Example 6 Acute Toxicity Test on Mice

Using a gastric probe, zeaxanthin (having a purity of 98.5%) was forcibly orally administered to each mouse (ICR male weighing 20~25 g, n=3) in an amount of 1000 or 2000 mg/kg. After the oral administration, the mice were reared in a cage for 7 days to observe the number of deaths and general conditions. An approximate median lethal dose ($LD_{50}$, in mg/kg) was estimated from the survival rate of the mice after observation. As a result, it was found that the

$LD_{50}$ of zeaxanthin exceeds 2000 mg/kg, which indicates that zeaxanthin is highly safe.

Preparation Example 1 (Powder of 5% Zeaxanthin)

| Zeaxanthin | 50 mg |
|---|---|
| Lactose | 950 mg |
| | Total 1000 mg |

Powder of 5% zeaxanthin was prepared by pounding crystals of zeaxanthin (having a purity of 98.5%) in a mortar, adding lactose in the mortar, and continuing pounding with a pestle until completely mixed.

Preparation Example 2 (Granules of 10% Zeaxanthin)

| Zeaxanthin | 300 mg |
|---|---|
| Lactose | 2000 mg |
| Starch | 670 mg |
| Gelatin | 30 mg |
| | Total 3000 mg |

Zeaxanthin (having a purity of 98.5%) was mixed and pounded with the same amount of starch in a mortar. Lactose and the remainder of the starch were added to and mixed with this mixture. A gelatin solution was separately prepared by adding 1 ml of purified water to 30 mg of gelatin, heating to dissolve the gelatin, and after cooling, adding 1 ml of ethanol to the solution while stirring. Granulates were prepared by adding the gelatin solution to the above mixture, kneading them together, granulating the resultant, and then drying.

Preparation Example 3 (Tablet comprising 5 mg Zeaxanthin)

| Zeaxanthin | 5 mg |
|---|---|
| Lactose | 62 mg |
| Starch | 30 mg |
| Talc | 2 mg |
| Magnesium Stearate | 1 mg |
| | Total 100 mg |

Tablets each comprising 5 mg of zeaxanthin were prepared in a mortar using the ingredients in amounts 20 times larger than those in the above composition. That is, 100 mg of zeaxanthin crystals (having a purity of 98.5%) were pounded, and lactose and starch were added to and mixed with zeaxanthin. Starch paste was added to this mixture, and the mixture was kneaded and granulated. After drying, talc and stearic acid were further mixed in, and tablets were produced by a conventional process.

Preparation Example 4 (Capsule comprising 5 mg Zeaxanthin)

| Zeaxanthin | 5 mg |
|---|---|
| Soya-bean oil | 155 mg |
| Beeswax | 20 mg |
| Fatty acid ester of glycerin | 20 mg |
| Gelatin (enclosing material) | 150 mg |
| | Total 100 mg |

155 g of soya-bean oil and 5 g of zeaxanthin (having a purity of 98.5%) were put in an emulsifying machine and mixed. To this mixture, 20 g of beeswax and 20 g of fatty acid ester of glycerin were added, and were uniformly dispersed by emulsification. Each capsule was formed by encapsulating 200 mg of this dispersion in 150 mg gelatin.

**Claims**

1.  Zeaxanthin for use as an active ingredient in a medicament to inhibit carcinogenesis.

2.  Zeaxanthin according to Claim 1, wherein zeaxanthin is extracted from an alga.

3.  Zeaxanthin according to Claim 2, wherein the alga is cyanobacterium.

4.  Zeaxanthin according to Claim 3, wherein the cyanobacterium is an alga belonging to the genus *Spirulina*.

5.  Use of the zeaxanthin of any one of Claims 1-4 for the manufacture of a medicament to inhibit carcinogenesis.

6.  Use of the zeaxanthin of any one of Claims 1-4 as an active ingredient for inhibiting carcinogenesis.

7.  An agent for inhibiting carcinogenesis, the agent comprising the zeaxanthin of any one of Claims 1-4.

8.  Use of the agent of Claim 7 for inhibiting carcinogenesis.

9.  Zeaxanthin for use as an active ingredient in a medicament to inhibit metastasis of tumor.

10.  Zeaxanthin according to Claim 9, wherein zeaxanthin is extracted from an alga.

11.  Zeaxanthin according to Claim 10, wherein the alga is cyanobacterium.

12.  Zeaxanthin according to Claim 11, wherein the cyanobacterium is an alga belonging to the genus *Spirulina*.

13.  Use of the zeaxanthin of any one of Claims 9-12 for the manufacture of a medicament to inhibit metastasis of tumor.

14.  Use of the zeaxanthin of any one of Claims 9-12 as an active ingredient for inhibiting metastasis of tumor.

15.  An agent for inhibiting metastasis of tumor, the agent comprising the zeaxanthin of any one of Claims 9-12.

16.  Use of the agent of Claim 15 for inhibiting carcinogenesis.

**European Patent**
**Office**

**PARTIAL EUROPEAN SEARCH REPORT**

which under Rule 45 of the European Patent Convention shall be considered, for the purposes of subsequent proceedings, as the European search report

**Application Number**

EP 98 10 9458

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | H.GERSTER: "Anticarcinogenic Effect of Common Carotenoids" INT.J.VIT.NUTR.RES., vol. 63, no. 2, 1993, pages 93-121, XP002082061 * abstract * | 1-16 | A61K31/045 |
| X | L. MILO ET AL.: "LUTEIN AND ZEAXANTHIN INHIBIT HUMAN BREAST CANCER CELL PROLIFERATION" FASEB J., vol. 12, no. 5, 20 March 1998, page A830 XP002082062 * abstract * | 1-12 | |
| X | WO 95 00130 A (THE HOWARD FOUNDATION) 5 January 1995 * claims 5,7,9 * | 1-16 | |
| X | WO 95 18605 A (MARIGEN S.A.) 13 July 1995 * claim 1 * | 1-16 | TECHNICAL FIELDS SEARCHED (Int.Cl.6) |
| | -/-- | | A61K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims

Claims searched completely :

Claims searched incompletely ·

Claims not searched :

Reason for the limitation of the search:

Although claims 6, 8, 14 and 16 are directed to a method of treatment of the human/animal body (Article 52(4) EPC), the search has been based on the alleged effects of the compound/composition.

| Place of search | Date of completion of the search | Examiner |
|---|---|---|
| THE HAGUE | 30 October 1998 | Theuns, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

**PARTIAL EUROPEAN SEARCH REPORT**

Application Number

EP 98 10 9458

| | DOCUMENTS CONSIDERED TO BE RELEVANT | | CLASSIFICATION OF THE APPLICATION (Int.Cl.6) |
|---|---|---|---|
| Category | Citation of document with indication, where appropriate, of relevant passages | Relevant to claim | |
| X | A. TSURUTA ET AL.: "Anti-tumor promoting effect of natural carotenoids, beta-cryproxanthin and zeaxanthin" PROCEEDINGS OF THE AMERICAN ASSOCIATION FOR CANCER RESEARCH ANNUAL MEETING, vol. 39, March 1998, page 288 XP002082063 * abstract * --- | 1-16 | |
| X | T.KATO ET AL.: "Cancer Prevention and Inhibition of Tumor Metastasis by Spirulina Components" DIC TECH. REV., no. 4, 1998, pages 49-54, XP002082697 * the whole document * --- | 1-16 | |
| X | D.L.MADHAVI ET AL.: "Isolation of bioactive constituents from Vaccinium myrtillus (bilberry) fruits and cell cultures" PLANT SCIENCE, vol. 131, no. 1, 1998, pages 95-103, XP002082698 * abstract * --- | 1-16 | **TECHNICAL FIELDS SEARCHED** (Int.Cl.6) |
| X | T.J.KING ET AL.: "Metabolites of dietary carotenoids as potential cancer preventive agents" PURE APPL. CHEM., vol. 69, no. 10, 1997, pages 2135-2140, XP002082064 * page 2139, last paragraph - page 2140 * --- -/-- | 1-16 | |

**European Patent Office**

## PARTIAL EUROPEAN SEARCH REPORT

Application Number

EP 98 10 9458

| | DOCUMENTS CONSIDERED TO BE RELEVANT | | CLASSIFICATION OF THE APPLICATION (Int.Cl.6) |
|---|---|---|---|
| Category | Citation of document with indication, where appropriate, of relevant passages | Relevant to claim | |
| X | H. GERSTER: "The 11th International Symposium on Carotenoids, Leiden, 18–23 August 1996" JOURNAL OF NUTRITIONAL & ENVIRONMENTAL MEDICINE, vol. 7, no. 3, September 1997, pages 197–201, XP002082065 * page 199, last paragraph - page 200, paragraph 1 * | 1-16 | |
| X | J.F.DORGAN ET AL.: "Relationships of serum carotenoids, retinol, alpha-tocopherol, and selenium with breast cancer risk: results from a prospective study in Columbia, Missouri (United States)" CANCER CAUSES CONTROL, vol. 9, no. 1, January 1998, pages 89–97, XP002082066 * abstract * | 1-16 | TECHNICAL FIELDS SEARCHED (Int.Cl.6) |
| X | Y. ITO ET AL.: "Relationship between Serum Xanthophyll Levels and the Consumption of Cigarettes, Alcohol or Foods in Healthy Inhabitants of Japan" INT.J.EPIDEMIOL., vol. 20, no. 3, September 1991, pages 615–620, XP002082067 * abstract * | 1-16 | |
| X | J.L. FREUDENHEIM ET AL.: "Premenopausal Breast Cancer Risk and Intake of Vegetables, Fruits, and Related Nutrients" J. NATL. CANCER INST., vol. 88, no. 6, 20 March 1996, pages 340–348, XP002082068 * abstract * | 1-16 | |

-/--

EPO FORM 1503 03.82 (P04C10)

**European Patent Office**  **PARTIAL EUROPEAN SEARCH REPORT**  Application Number

EP 98 10 9458

| | **DOCUMENTS CONSIDERED TO BE RELEVANT** | | **CLASSIFICATION OF THE APPLICATION (Int.Cl.6)** |
|---|---|---|---|
| Category | Citation of document with indication, where appropriate, of relevant passages | Relevant to claim | |
| X | J.L.FREUDENHEIM ET AL.: "INDIVIDUAL CAROTENOIDS AND TOTAL VEGETABLES IN THE EPIDEMIOLOGY OF PREMENOPAUSAL BREAST CANCER" FASEB J., vol. 9, no. 3, 1995, page A579 XP002082069 * abstract * | 1-16 | |
| X | M. TSUSHIMA ET AL.: "Inhibitory Effect of Natural Carotenoids on Epstein-Barr Virus Activation Activity of a Tumor Promotor in Raji Cells. A Screening Study for Anti-tumor Promotors" BIOL. PHARM. BULL., vol. 18, no. 2, February 1995, pages 227-233, XP002082070 * page 232, right-hand column * | 1-16 | **TECHNICAL FIELDS SEARCHED (Int.Cl.6)** |
| X | F.KHACHIK ET AL.: "Lutein, Lycopene, and Their Oxidative Metabolites in Chemoprevention of Cancer" J.CELL.BIOCHEM., no. Supplement 22, 1995, pages 236-246, XP002082071 * abstract * | 1-16 | |
| X | C. LA VECCHIA ET AL.: "Role of various carotenoids in the risk of breast cancer" INT.J.CANCER, vol. 75, no. 3, 30 January 1998, pages 482-483, XP002082072 * the whole document * | 1-16 | |

-/--

EPO FORM 1503 03.82 (P04C10)

**European Patent Office**

## PARTIAL EUROPEAN SEARCH REPORT

**Application Number**

EP 98 10 9458

| | DOCUMENTS CONSIDERED TO BE RELEVANT | | CLASSIFICATION OF THE APPLICATION (Int.Cl.6) |
|---|---|---|---|
| Category | Citation of document with indication, where appropriate, of relevant passages | Relevant to claim | |
| X | S. ZHANG ET AL.: "Measurement of retinoids and carotenoids in breast adipose tissue and a comparison of concentrations in breast cancer cases and control subjects" AM.J.CLIN.NUTR., vol. 66, no. 3, September 1997, pages 626-632, XP002082073 * abstract * | 1-16 | |
| X | L.A.MOONEY ET AL.: "Application of Molecular Epidemiology to Lung Cancer Chemoprevention" J. CELL. BIOCHEM. SUPPL., vol. 25S, 1996, pages 63-68, XP002082074 * abstract * | 1-16 | TECHNICAL FIELDS SEARCHED (Int.Cl.6) |
| X | P. DIMASCIO ET AL.: "Carotenoids, tocopherols and thiols as biological singlet molecular oxygen quenchers" BIOCHEM.SOC.TRANS., vol. 18, no. 6, December 1990, pages 1054-1056, XP002082075 * abstract * | 1-16 | |
| X | J.L.SCHWARTZ ET AL.: "A Cyanobacteria Extract and beta-Carotene Stimulate an Antitumor Host Response Against an Oral Cancer Cell Line" PHYTOTHER.RES., vol. 3, no. 6, 1989, pages 243-248, XP002082076 * the whole document * | 1-16 | |

-/--

EPO FORM 1503 03.82 (P04C10)

European Patent
Office

**PARTIAL EUROPEAN SEARCH REPORT**

Application Number

EP 98 10 9458

| | DOCUMENTS CONSIDERED TO BE RELEVANT | | CLASSIFICATION OF THE APPLICATION (Int.Cl.6) |
|---|---|---|---|
| Category | Citation of document with indication, where appropriate, of relevant passages | Relevant to claim | |
| X,P | H. NISHINO: "Cancer prevention by carotenoids" MUTATION RESEARCH, vol. 402, no. 1-2, 18 June 1998, pages 159-163, XP002082077 * abstract * --- | 1-16 | |
| X,P | CHEMICAL ABSTRACTS, vol. 129, no. 13, Columbus, Ohio, US; abstract no. 166188z, XP002082078 * abstract * & JP 10 212228 A (DAINIPPON INK AND CHEMICALS, INC.) 11 August 1998 ----- | 1-16 | |
| | | | TECHNICAL FIELDS SEARCHED (Int.Cl.6) |

EPO FORM 1503 03.82 (P04C10)

**ANNEX TO THE EUROPEAN SEARCH REPORT
ON EUROPEAN PATENT APPLICATION NO.**                EP 98 10 9458

This annex lists the patent family members relating to the patent documents cited in the above-mentioned European search report.
The members are as contained in the European Patent Office EDP file on
The European Patent Office is in no way liable for these particulars which are merely given for the purpose of information.

30-10-1998

| Patent document cited in search report | | Publication date | Patent family member(s) | | Publication date |
|---|---|---|---|---|---|
| WO 9500130 | A | 05-01-1995 | AU | 7005694 A | 17-01-1995 |
| | | | GB | 2280110 A,B | 25-01-1995 |
| | | | ZA | 9404633 A | 25-10-1995 |
| WO 9518605 | A | 13-07-1995 | CH | 685325 A | 15-06-1995 |
| | | | EP | 0719136 A | 03-07-1996 |

EPO FORM P0459

For more details about this annex : see Official Journal of the European Patent Office, No. 12/82